# EUROPÄISCHE PATENTSCHRIFT

(11) **EP 2 602 000 B1**
(45) Veröffentlichungstag und Bekanntmachung des Hinweises auf die Patenterteilung: **01.06.2016**
(21) Anmeldenummer: 12192386.6
(22) Anmeldetag: 13.11.2012
(51) Int. Cl.: A61N 1/05, A61N 1/08

(54) **Implantierbare Elektrodenleitung**
Implantable electrode lead
Ligne d'électrodes implantables

(30) Priorität: 08.12.2011 US 201161568182 P
(43) Veröffentlichungstag der Anmeldung: 12.06.2013
(73) Patentinhaber: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Galander, Marcus

(56) Entgegenhaltungen:
- US-A1- 2008 161 886
- US-A1- 2011 087 299
- US-A1- 2011 208 280
- US-A1- 2011 270 362

## Beschreibung

Die Erfindung betrifft eine implantierbare längsgestreckte Elektrodenleitung mit einem Elektrodenkörper, wenigstens einem mit dem Elektrodenkörper verbundenen Elektrodenpol, einem Anschluss und einer elektrischen Zuleitung, die elektrisch mit dem Elektrodenpol verbunden ist und die sich von dem Elektrodenpol zu dem Anschluss an einem proximalen Ende der Elektrodenleitung erstreckt.

Derartige Elektrodenleitungen werden beispielsweise an implantierbare Herzstimulatoren wie Herzschrittmacher, Kardioverter, Defibrillatoren oder dergleichen angeschlossen, können aber auch als Mapping Katheter zur Diagnose oder zur Neurostimulation dienen.

Solche Elektrodenleitungen haben den Nachteil, dass sich ihr elektrischer Leiter in einem Kernspintomografen erwärmen kann, weil die im Kernspintomografen herrschenden wechselnden Magnetfelder in dem elektrischen Leiter nicht unbeachtliche elektrische Ströme induzieren. Deshalb können Herzschrittmacherpatienten heutzutage in der Regel nicht oder nur eingeschränkt in einem Kernspintomografen untersucht werden.

An implantierbaren Herzschrittmachern oder Defibrillatoren sind nämlich typischerweise wenigstens eine Stimulationselektrodenleitung angeschlossen, die an ihrem proximalen, zum Anschluss an den Herzschrittmacher oder Defibrillator vorgesehenen Ende einen standardisierten elektrischen Anschluss aufweist und an ihrem distalen, zur Platzierung im Herzen vorgesehenen Ende einen oder mehrere Elektrodenpole aufweist. Ein solcher Elektrodenpol dient zur Abgabe elektrischer Impulse, beispielsweise an das Gewebe (Myokard) des Herzens oder zum Abfühlen elektrischer Felder, um im Rahmen des sogenannten Sensings eine Aktivität, beispielsweise eine Herzaktivität, abfühlen zu können.

Zu diesen Zwecken bilden Elektrodenpole typischerweise elektrisch leitende Oberflächenabschnitte einer Elektrodenleitung. Elektrodenpole sind typischerweise als Ringelektrode in Form eines Rings um die Elektrodenleitung oder in Form einer Spitzen- oder Tippelektrode am distalen Ende der Elektrodenleitung vorgesehen.

Die Elektrodenpole sind über eine oder mehrere elektrische Zuleitungen mit Kontakten des elektrischen Anschlusses der Elektrodenleitung an deren proximalem Ende elektrisch leitend verbunden. Somit verlaufen zwischen den Kontakten des elektrischen Anschlusses die Elektrodenleitungen an deren proximalem Ende und den Elektrodenpolen am distalen Ende der Elektrodenleitung ein oder mehrere elektrische Zuleitungen, die einen oder mehrere der Elektrodenpole mit einem oder mehreren der Kontakte elektrisch verbinden.

Dokument US2008/161886 offenbart eine implantierbare Elektrodenleitung nach der Präambel des Anspruchs 1.

Solche Zuleitungen enthalten für die Funktionen der jeweiligen Elektrodenleitung erforderliche elektrische Leiter und sind daher der Gefahr ausgesetzt, dass in ihnen durch äußere Wechselmagnetfelder elektrische Ströme induziert werden, die beispielsweise zu einer unerwünschten Erwärmung der Zuleitungen oder der mit ihr verbundenen Elektrodenpole führen können oder die zur Abgabe entsprechender Ströme über die Elektrodenpole an umgebendes Gewebe und damit zu einer Erwärmung des umgebenden Gewebes führen können.

Ziel der Erfindung ist es, eine Elektrodenleitung zu schaffen, die eine geringere Wärmeentwicklung als herkömmliche Elektrodenleitungen in einer MRT-Umgebung erwarten lässt.

Erfindungsgemäß wird dieses Ziel durch eine implantierbare Elektrodenleitung gemäß Anspruch 1 erreicht.

Bevorzugt weist die implantierbare Elektrodenleitung eine äußere Hülle gemäß Anspruch 2 auf.

Die Hülle nimmt somit nicht die Form des Zuleitungsabschnitts ein, wenn dieser seine veränderte Form einnimmt.

Der beabsichtigte Effekt der Reduktion der Wärmeentwicklung dieser Elektroden beruht auf der Verstimmung der für die MRT-Felder resonanten Antenneneigenschaften der Elektrodenleitung, entweder durch eine Längen- bzw. Geometrievariation und/oder das Hinzufügen einer Induktivität.

Die Hülle ist im Bereich des formveränderlichen Zuleitungsabschnitts vorzugsweise dehnbar und formveränderlicher Zuleitungsabschnitt und Hülle sind so ausgebildet, dass sich die Hülle bei Formveränderung des formveränderlichen Zuleitungsabschnitts radial ausdehnt.

Es ergibt sich eine MRT-kompatible Elektrodenleitung, die nach der Implantation ganz oder teilweise eine gewendelte Form einnimmt und somit eine für die MRT-HF-Felder schlecht abgestimmte Antenne darstellt und damit die MRT-induzierte Wärmeentwicklung reduziert.

Die Erfindung schließt die Erkenntnis ein, dass bislang zwar verschiedenste Bauformmodifikationen und auch die Integration von elektronischen Bauelementen in Elektrodenleitungen bekannt sind, um die MRT-induzierte Wärmeentwicklung zu reduzieren. Jedoch bedürfen viele der oben genannten Lösungen zur Reduktion der MRT-induzierten Wärmeentwicklung in Elektrodenleitungen zusätzlicher Konstruktionsmerkmale, die den Gesamtaufbau einer Elektrodenleitung wesentlich komplexer machen und Fügestellen hinzufügen, so dass die Zuverlässigkeit dieser Elektrodenleitungen gegenüber herkömmlichen Elektrodenleitungen wahrscheinlich abnehmen wird.

Gemäß einer bevorzugten Ausführungsform ist die veränderte Form des formveränderlichen Zuleitungsabschnitts nach Implantation der Elektrodenleitung eine Helixform. Diese ist geeignet, dem formveränderlichen Zuleitungsabschnitt eine gewünschte Induktivität zu verleihen und kann darüber hinaus auch einer Fixierung der Elektrodenleitung nach Implantation dienen, indem nicht nur die Zuleitung selbst, sondern auch die Außenform der Elektrodenleitung einschließlich äußerer Hülle im Bereich des formveränderlichen Zuleitungsabschnitts eine Helixform annimmt, die sich z.B. an die Wand eines Blutgefäßes anlegt.

Vorzugsweise weist die Helixform wenigstens 15 Windungen, insbesondere mehr als 30 Windungen, auf. Die einzelnen Windungen der Helixform haben vorzugsweise einen Abstand voneinander. Die veränderte Form bewirkt vorzugsweise eine Induktivität von mehr als 1 µH.

Die veränderte Form der Zuleitung ist voreingeprägt. Hierzu kann die Elektrodenleitung aus einem Memory-Metall so hergestellt sein, dass sie nach Überschreiten einer Springtemperatur die veränderte Form einnimmt. Alternativ oder zusätzlich kann die Elektrodenleitung vorgeformt und beim Implantieren mittels einer steiferen entfernbaren Einführhilfe elastisch vorgespannt sein, so dass die Zuleitung nach Entfernen der Einführhilfe auf der Vorspannung die veränderte Form einnimmt. Hierbei ist die Einführhilfe vorzugsweise ein Hüllkatheter oder ein Stylet.

Zum Erzielen einer voreingeprägten Form kann bei einer Zuleitung, die einen elektrischen Leiter umfasst, vorgesehen sein, dass wenigstens der elektrische Leiter im Bereich des formveränderlichen Zuleitungsabschnitts von einem Mantel umschlossen ist, dem veränderte Form der Zuleitung voreingeprägt ist. Der Mantel ist hierbei vorzugsweise von einem Memorymaterial gebildet. Außerdem ist es bevorzugt, wenn der Mantel von isolierendem Kunststoff gebildet ist.

Vorzugsweise hat der formveränderliche Zuleitungsabschnitt nach Formänderung einen Durchmesser von mehr als 8 mm oder, wenn möglich, mehr als 10mm. Dies erhöht die Induktivität des Zuleitungsabschnitts.

Der formveränderliche Zuleitungsabschnitt ist vorzugsweise in der Nähe eines distalen Endes der Elektrodenleitung vorgesehen, kann sich aber auch an einem proximalen Ende der Elektrodenleitung befinden oder in deren Mitte. Es ist vorzugsweise nur ein formveränderlicher Zuleitungsabschnitt vorgesehen, es können aber auch deren mehrerer vorgesehen sein.

Das Ziel der Erfindung wird somit mit einer temporär oder dauerhaft implantierbaren Elektroden- oder Sensorleitung mit mindestens einer elektrischen Zuleitung erreicht, die so aufgebaut ist, dass sich ein oder mehrere Teile der oder die gesamte elektrische Zuleitung nach der Implantation spulenförmig oder gekräuselt ausrichtet.

Zur einfachen Explantation der Elektrodenleitung ist die Formveränderung reversibel und kann z.B. durch das Einführen eines steifen Stylets o.ä. wenigstens temporär rückgängig gemacht werden.

Die Erfindung soll nun anhand eines Ausführungsbeispiels mit Bezug auf die Figuren näher erläutert werden. Von den Figuren zeigt:
- Fig. 1: eine erfindungsgemäße Elektrodenleitung vor Implantation;
- Fig. 2: die Elektrodenleitung aus Figur 1 nach Implantation; und
- Fig. 3 bis 12: weitere Beispiele für einen Zuleitungsabschnitt mit veränderter Form.

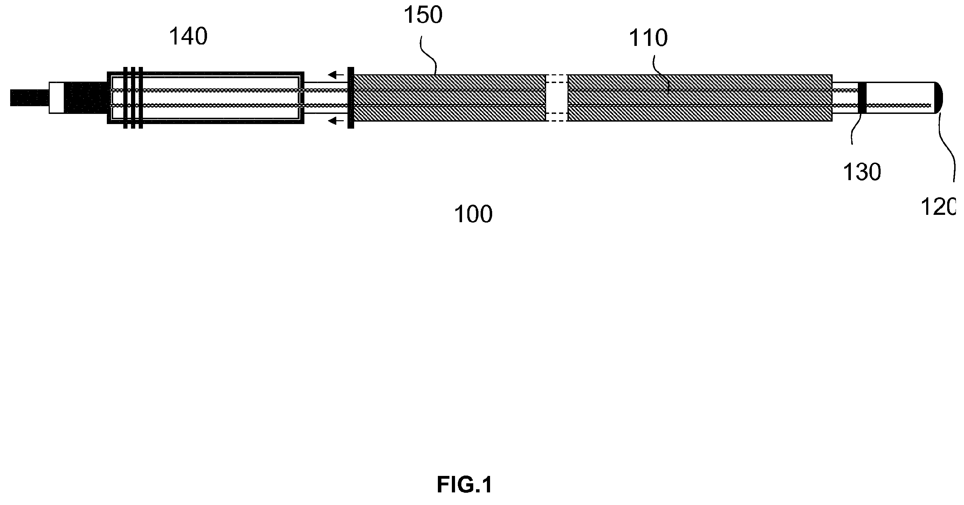

In Figur 1 ist eine erfindungsgemäße Elektrodenleitung (100) vor Implantation dargestellt. Die Elektrodenleitung (100) besitzt eine isolierte Zuleitung (110), einen bipolaren Elektrodenpol (Tip: 120, Ring 130) und einen entsprechenden Anschluss-Stecker (140) und eine temporär zu verwendende Einführhilfe (150), um bei Implantation eine an sich gestreckte Form herzustellen. Diese Einführhilfe (150) ist im dargestellten Beipiel als Einführschleuse bzw. Schlauch über der Elektrodenleitung ausgeführt. Alternativ kann aber auch ein steifes Stylet oder ein Mandrin verwendet werden.

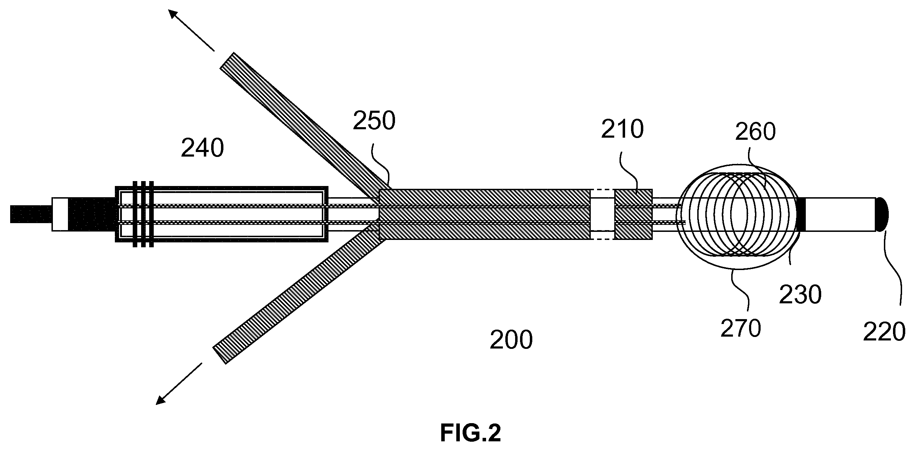

In Figur 2 ist die Elektrodenleitung bei Abschluss der Implantation dargestellt. Zusätzlich zu den in Figur 1 genannten Merkmalen ist jetzt ein formveränderlicher Zuleitungsabschnitt (260) der Elektrodenleitung skizziert. In diesem Ausführungsbeispiel bildet der formveränderliche Zuleitungsabschnitt im distalen Bereich der Elektrodenleitung eine Spule mit einem Durchmesser von 3 - 15 mm. Die veränderte Form des formveränderlichen Zuleitungsabschnitts (260) ist entweder durch eine Vorformung eines Mantels der Zuleitung und/oder eines elektrischen Leiters der Zuleitung oder durch ein Shape Memory Material (Polymer oder Metall) hervorgerufen, das durch die Körperwärme oder einen zusätzlichen Energieeintrag (Wärme, Licht) die Ausformung realisiert.

Die helixförmige Wendel kann dabei entstehen, indem die gesamte Elektrodenleitung samt in ihr befindlichem Zuleitungsabschnitt gewendelt wird oder aber bevorzugt eine Wendel der Zuleitung innerhalb der äußeren Hülle der Elektrodenleitung entsteht, wobei sich dann die äußere Hülle (270) ballonförmig aufweitet. Letzteres ist in Figur 2 angedeutet.

Die Induktivität der vom formveränderlichen Zuleitungsabschnitt gebildeten Helix-Wendel beträgt im gezeigten Beispiel etwa 1 µH, d.h. die Wendel hat ca. 30 Windungen auf einer Länge von 10 cm mit einem Durchmesser von 1,2 cm. Zusammen mit der Gewebeimpedanz ergibt sich so ein Spannungsteiler, die die Elektrodenerwärmung mindestens um den Faktor 2 reduziert.

Die so gebildete Elektrodenleitung ist MRT-tauglich und besitzt an den qualitätsrelevanten Fügestellen keine Abweichungen von herkömmlichen Elektrodenleitungen. Ebenso werden keine zusätzlichen elektronischen Bauelemente in der Elektrode benötigt.

Ausführungen zur veränderten Form des Zuleitungsabschnitts nach Implantation:
Die Kräuselung (Stauchung, Unterbringung einer größeren Länge auf kleinem Raum) erfolgt bei oder nach Implantation. Beispielsweise nimmt der formveränderliche Zuleitungsabschnitt bei Entfernung eines Mandrins eine vorgeformte/voreingeprägte Form an. Alternativ oder zusätzlich findet dies durch Wärmeeinfluss statt (Memory shape) - d.h. durch die Eigenwärme des Körpers oder Erwärmen des Mandrins.

Die Kräuselung ist bevorzugt im distalen Bereich, ggf. zusätzlich im proximalen Bereich oder auch nur im proximalen Bereich, ausgebildet.

In einer weiteren Ausführung sind mehrere gekräuselte Bereiche entlang der Elektrodenleitung ausgeprägt. Diese sind bevorzugt an die lokalen anatomischen Gegebenheiten angepasst, z.B. in der oberen Hohlvene eine Form, im Vorhof eine zweite Form und im Ventrikel eine dritte Form.

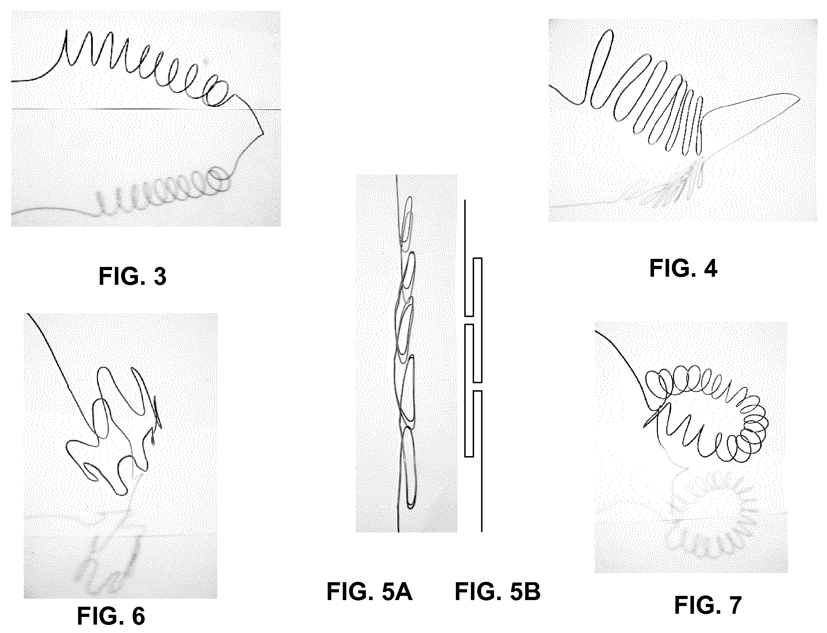

Die veränderte Form kann verschiedene Ausprägungen haben:
Diese Formen gehen im Wesentlichen von zwei Grundformen aus und darauf aufbauend (durch Kombinatorik/Verschachtelung) entsteht eine Vielfalt weiterer Formen, wie sie in den Figuren 3 bis 12 abgebildet sind.

Die Grundformen sind im Wesentlichen:
Helix, siehe Figur 3, und
Mäander, siehe Figur 4.

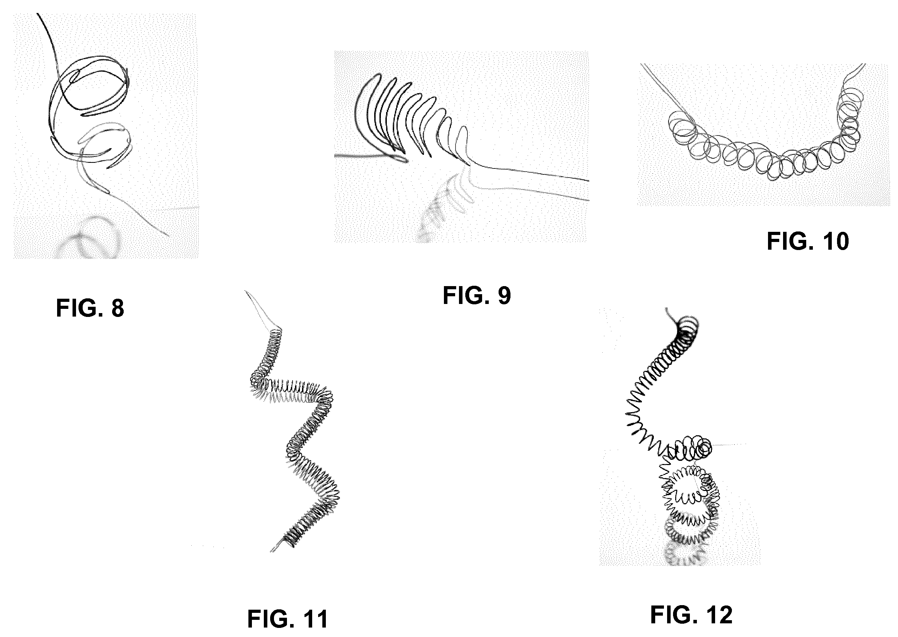

Varianten des Mäander und der Helix sind in Figuren 5 bis 12 dargestellt, nämlich:
Mäander als Torus, siehe Figur 6;
Helix als Torus, siehe Figur 7;
Mäander als Helix, siehe Figur 8;
Mäander geformt, siehe Figur 9;
Helix flachgedrückt, siehe Figur 10;
Helix als Mäander, siehe Figur 11;
Helix als Helix, siehe Figur 12.

Die Elektrodenleitungen für diese gekräuselten Ausführungen haben bevorzugt eine Dicke von weniger als 5 F.

## Patentansprüche

1. Implantierbare längsgestreckte Elektrodenleitung mit einem Elektrodenkörper, wenigstens einem mit dem Elektrodenkörper verbundenen Elektrodenpol, einem Anschluss und einer elektrischen Zuleitung, die elektrisch mit dem Elektrodenpol verbunden ist und die sich von dem Elektrodenpol zu dem Anschluss an einem proximalen Ende der Elektrodenleitung erstreckt,
**dadurch gekennzeichnet,**
**dass** die elektrische Zuleitung ausgebildet ist, nach Implantation der Elektrodenleitung in einem formveränderlichen Zuleitungsabschnitt eine solche veränderte Form einzunehmen, die in dem formveränderlichen Zuleitungsabschnitt nach Verformung eine höhere Induktivität als vor der Verformung bewirkt, wobei die Induktivität mindestens 0,1 µH beträgt, und dass die veränderte Form der Zuleitung voreingeprägt ist.

2. Implantierbare längsgestreckte Elektrodenleitung gemäß Anspruch 1,
**dadurch gekennzeichnet,**
**dass** die implantierbare längsgestreckte Elektrodenleitung eine äußere Hülle aufweist, innerhalb derer die Zuleitung angeordnet ist, und die Zuleitung außerdem so ausgebildet ist und innerhalb der Hülle so angeordnet ist, dass die Zuleitung in ihrem formveränderlichen Zuleitungsabschnitt relativ zur äußeren Hülle und innerhalb der äußeren Hülle formveränderlich ist.

3. Elektrodenleitung gemäß Anspruch 2, **dadurch gekennzeichnet, dass** die Hülle im Bereich des formveränderlichen Zuleitungsabschnitts dehnbar ist und formveränderlicher Zuleitungsabschnitt und Hülle so ausgebildet sind, dass sich die Hülle bei Formveränderung des formveränderlichen Zuleitungsabschnitts radial ausdehnt.

4. Elektrodenleitung gemäß einem der Ansprüche 1 bis 3, **dadurch gekennzeichnet, dass** die veränderte Form eine Helixform ist.

5. Elektrodenleitung gemäß Anspruch 4, **dadurch gekennzeichnet, dass** die Helixform wenigstens 15 Windungen aufweist.

6. Elektrodenleitung gemäß Anspruch 4 oder 5, **dadurch gekennzeichnet, dass** einzelne Windungen der Helixform einen Abstand voneinander haben.

7. Elektrodenleitung gemäß einem der Ansprüche 1 bis 6, **dadurch gekennzeichnet, dass** die veränderte Form eine Induktivität von mehr als 1 µH bewirkt.

8. Elektrodenleitung gemäß Anspruch 1, **dadurch gekennzeichnet, dass** die Elektrodenleitung aus einem Formgedächtnis-Metall so hergestellt ist, dass sie nach Überschreiten einer Springtemperatur die veränderte Form einnimmt.

9. Elektrodenleitung gemäß Anspruch 1, **dadurch gekennzeichnet, dass** die Elektrodenleitung vorgeformt und beim Implantieren mittels einer steiferen entfernbaren Einführhilfe elastisch vorgespannt ist, so dass die Zuleitung nach Entfernen der Einführhilfe auf der Vorspannung die veränderte Form einnimmt.

10. Elektrodenleitung gemäß Anspruch 9, **dadurch gekennzeichnet, dass** die Einführhilfe ein Hüllkatheter oder ein Stylet ist.

11. Elektrodenleitung gemäß einem der Ansprüche 8 bis 10, **dadurch gekennzeichnet, dass** die Zuleitung einen elektrischen Leiter umfasst, der wenigstens im Bereich des formveränderlichen Zuleitungsabschnitts von einem Mantel umschlossen ist, dem die veränderte Form der Zuleitung voreingeprägt ist.

12. Elektrodenleitung gemäß Anspruch 11, **dadurch gekennzeichnet, dass** der Mantel von einem Formgedächtnismaterial gebildet ist.

13. Elektrodenleitung gemäß Anspruch 11 oder 12, **dadurch gekennzeichnet, dass** der Mantel von isolierendem Kunststoff gebildet ist.

14. Elektrodenleitung gemäß einem der Ansprüche 1 bis 13, **dadurch gekennzeichnet, dass** der formveränderliche Zuleitungsabschnitt nach Formänderung einen Durchmesser von mehr als 8 mm hat.

## Claims

1. An implantable elongate electrode lead comprising an electrode body, at least one electrode pole connected to the electrode body, a connector, and an electric supply line which is electrically connected to the electrode pole and extends from the electrode pole to the connector at a proximal end of the electrode lead,
**characterised in that**
the electric supply lead is configured to take such an altered shape in a deformable supply line portion after implantation of the electrode lead, such altered shape causing an inductance that is higher in the deformable supply line portion after deformation than before deformation, wherein the inductance is at least 0.1 µH, and **in that** the altered shape of the supply lead is pre-impressed.

2. The implantable elongate electrode lead according to claim 1,
**characterised in that**
the implantable elongate electrode lead has an outer sleeve within which the supply line is disposed, and the supply line is additionally configured and disposed inside the sleeve such that the supply line, in the deformable supply line portion thereof, is deformable relative to the outer sleeve and inside the outer sleeve.

3. The electrode lead according to claim 2, **characterised in that** the sleeve is expandable in the region of the deformable supply line portion, and the deformable supply line portion and sleeve are configured such that the sleeve expands radially upon deformation of the deformable supply line portion.

4. The electrode lead according to any one of claims 1 to 3, **characterised in that** the altered shape is a helix.

5. The electrode lead according to claim 4, **characterised in that** the helix comprises at least 15 turns.

6. The electrode lead according to claim 4 or 5, **characterised in that** individual turns of the helix are distanced from one another. □

7. The electrode lead according to any one of claims 1 to 6, **characterised in that** the altered shape causes an inductance of more than 1 µH.

8. The electrode lead according to claim 1, **characterised in that** the electrode lead is produced from a shape-memory metal, such that it takes the altered shape after a transition temperature is exceeded.

9. The electrode lead according to claim 1, **characterised in that** the electrode lead is pre-formed and, upon implantation, is elastically pre-loaded by means of an insertion aid that is stiffer and removable, such that the supply line takes the altered shape after removal of the insertion aid exerting the pre-load.

10. The electrode lead according to claim 9, **characterised in that** the insertion aid is a sleeve catheter or a stylet.

11. The electrode lead according to any one of claims 8 to 10, **characterised in that** the supply line comprises an electric conductor which is enclosed by a jacket at least in the region of the deformable supply line portion, upon which jacket the altered shape of the supply line is pre-impressed.

12. The electrode lead according to claim 11, **characterised in that** the jacket is formed of a shape-memory material.

13. The electrode lead according to claim 11 or 12, **characterised in that** the jacket is formed of an insulating plastic.

14. The electrode lead according to any one of claims 1 to 13, **characterised in that** the deformable supply line portion has a diameter of more than 8 mm after deformation.

## Revendications

1. Ligne d'électrode implantable rallongée avec un corps d'électrode, au moins un pôle d'électrode connecté avec le corps d'électrode, une connexion et une ligne électrique, qui est reliée électriquement avec le pôle d'électrode et qui s'étend du pôle d'électrode jusqu'à la connexion à une extrémité proximale de la ligne d'électrode,
**caractérisée en ce**
**que** la ligne électrique est conçue pour adopter une forme modifiée après l'implantation de la ligne d'électrode dans une portion d'alimentation déformable telle qu'elle provoque une inductance plus élevée dans la portion d'alimentation déformable après la transformation qu'avant la transformation, où l'inductance s'élève à au moins 0,1 µH, et que la forme modifiée de l'alimentation est pré-moulée.

2. Ligne d'électrode implantable rallongée selon la revendication 1,
**caractérisée en ce**
**que** la ligne d'électrode implantable rallongée présente une gaine extérieure, à l'intérieur de laquelle est disposée l'alimentation, et l'alimentation est en outre conçue de telle manière et agencée à l'intérieur de la gaine que l'alimentation est déformable dans sa portion d'alimentation déformable par rapport à la gaine extérieure et à l'intérieur de la gaine extérieure.

3. Ligne d'électrode selon la revendication 2, **caractérisée en ce que** la gaine est étirable dans la zone de la portion d'alimentation déformable et que la portion d'alimentation déformable et la gaine sont conçues de telle sorte que la gaine s'étire radialement lors d'une déformation de la portion d'alimentation déformable.

4. Ligne d'électrode selon l'une des revendications 1 à 3, **caractérisée en ce que** la forme modifiée est une forme hélicoïdale.

5. Ligne d'électrode selon la revendication 4, **caractérisée en ce que** la forme hélicoïdale présente au moins 15 enroulements.

6. Ligne d'électrode selon les revendications 4 ou 5, **caractérisée en ce que** des enroulements individuels de la forme hélicoïdale ont un espace entre eux.

7. Ligne d'électrode selon l'une des revendications 1 à 6, **caractérisée en ce que** la forme modifiée provoque une inductance supérieure à 1 µH.

8. Ligne d'électrode selon la revendication 1, **caractérisée en ce que** la ligne d'électrode est fabriquée dans un métal à mémoire de forme de sorte qu'elle adopte la forme modifiée après dépassement d'une température de transformation.

9. Ligne d'électrode selon la revendication 1, **caractérisée en ce que** la ligne d'électrode est préformée et est précontrainte élastiquement au moyen d'un accessoire d'insertion plus rigide pouvant être enlevé de sorte que l'alimentation adopte la forme modifiée sur la précontrainte après l'enlèvement de l'accessoire d'insertion.

10. Ligne d'électrode selon la revendication 9, **caractérisée en ce que** l'accessoire d'insertion est un cathéter gainant ou un stylet.

11. Ligne d'électrode selon l'une des revendications 8 à 10, **caractérisée en ce que** l'alimentation comprend un conducteur électrique qui est enfermé dans une gaine, dont la forme modifiée de l'alimentation est pré-moulée, au moins dans la zone de la portion d'alimentation déformable.

12. Ligne d'électrode selon la revendication 11, **caractérisée en ce que** la gaine est formée par un matériau à mémoire de forme.

13. Ligne d'électrode selon les revendications 11 ou 12, **caractérisée en ce que** la gaine est formée dans une matière synthétique isolante.

14. Ligne d'électrode selon l'une des revendications 1 à 13, **caractérisée en ce que** la portion d'alimentation déformable possède un diamètre supérieur à 8 mm après la déformation.
